# EUROPEAN PATENT SPECIFICATION

(11) **EP 2 010 276 B1**
(45) Date of publication and mention of the grant of the patent: **22.01.2014**
(21) Application number: 07756084.5
(22) Date of filing: 26.04.2007
(51) Int. Cl.: A61N 1/30

(54) **MULTI-STAGE MICROPORATION DEVICE**
MEHRFACHMODUS-MIKROPORATIONSVORRICHTUNG
DISPOSITIF DE MICROPORATION MULTI-ÉTAGÉE

(30) Priority: 26.04.2006 US 794979 P
(43) Date of publication of application: 07.01.2009
(73) Proprietor: Covidien LP, Mansfield, MA 02048 (US)
(72) Inventor: CHELAK, Todd, M., Westborough, MA 01581 (US)
(74) Representative: Gray, James
(86) International application number: PCT/US2007/010185
(87) International publication number: WO 2007/127339

(56) References cited:
- WO-A2-2006/004595
- WO-A2-2006/004595

## Description

### CROSS REFERENCE TO RELATED APPLICATION:

The present application claims the benefit of priority to U.S. Provisional Patent Application Serial No. 601794,979 filed on April 26, 2006 entitled "MULTI-STAGE MICROPORATION DEVICE" by Todd Chelak.

### BACKGROUND

### 1. Technical Field

The present disclosure is directed to a device for a transdermal delivery of a substance to a patient and, in particular, to a plural or multi-stage microporation device that delivers a substance transdermally to a patient, or extracts a substance transdermally from the patient.

### 2. Description of the Related Art

Transdermal delivery of drugs has been known for a number of years. The outermost layer of the skin is called the stratum corneum, which forms the top layer of the epidermis. Below the stratum corneum is the viable or remaining epidermis. Below the epidermis is the dermis layer of the skin. Known transdermal delivery of drugs has been accomplished with a patch or reservoir having an active agent therein. Once the outermost layer of the skin or stratum corneum is breached, such as by the creation of a number of micropores, the active agent is introduced through the stratum corneum, through the viable epidermis and into the dermis where, for example, the active agent can enter the blood stream at a measured rate without destroying or weakening the drug in the gastrointestinal tract or the stomach.

However, problems occur in that the transdermal rate of entry of the active agents through the skin largely depends on the molecular size and type of active agent being delivered and the geometry of the intercellular pathway through the stratum corneum. Several methods are known for enhancing or enlarging the pathway through the stratum corneum or more particularly, for creating micropores through the stratum corneum. Often if the micropore is not shaped or sized properly or if the micropore has an insufficient depth the active agent cannot reliably enter the bloodstream or be exposed to antigen presenting cells within the epidermis. Moreover, if the micropore is not shaped or sized properly, or if the micropore has an insufficient depth the active agent cannot enter the bloodstream at a sufficient rate for therapeutic effects of the active agent to take effect or cannot be properly exposed to antigen presenting cells for an effective immune response to occur. Further, pain and undesirable skin effects often correlated with the overall depth of the micropore due, in part, to undesirable increases in the width of the micropore. Therefore, a need exists for a microporation device that functions in a stage-like manner to initially form the micropore with an initial depth and then subsequently increase the initial depth and/or produce an improvement in at least one relevant physiological property of the micropore with a second disruptive event.

Document WO-A-2006/004595 discloses a device according to the preamble of claim 1.

### SUMMARY

The present disclosure relates to a thermal treatment device for forming a micropore in a barrier and includes at least one micro-heater having a base end and at least one post defined therein. The post includes an interior volume for housing an ablation material therein. The micro-heater is operatively associated with a power supply component configured to supply energy to the micro-heater to activate the ablation material such that the ablation material expands in response to the energy to mechanically puncture the stratum corneum to create a micropore having a first depth. The micro-heater is subsequently activated to enlarge the micropore to a second depth and/or initiate a tissue response by introducing a chemical into the micropore or ablating surrounding tissue. A chemical reaction may also stimulate a tissue response. The subsequent activation of the ablation material may also involve further heating the ablation material or may include a chemical reaction which enlarges the depth of the micropore.

In one envisioned embodiment, the first depth is about ten microns to about thirty microns and the second depth is greater than about thirty microns. In another envisioned embodiment, the first depth is in a range of about ten microns to about thirty microns and the second depth is sufficient to extend beyond thirty microns into the viable epidermis.

In one particular embodiment, the ablation material includes ethanol. In yet another embodiment, a plurality of ablation materials having different thermal conductivities may be utilized to create micropores of varying depths, to create micropores in stages by varying the depth of the micropores with each subsequent activation and/or inducing tissue stimulation by creating a reaction with the surrounding tissue.

In still yet another embodiment, the micro-heater includes an insulator material which prevents excessive heating (above about 100 degrees Centigrade) of the surrounding tissue during activation of the ablation material.

The present disclosure also relates to a thermal treatment device for forming a micropore in a barrier which includes one or more micro-heaters each having a base end and at least one post defined therein, the post including an interior volume for housing an ablation material. The micro-heater is adapted to connect to a power supply component which supplies energy to the micro-heater to activate the ablation material such that the ablation material expands in response to the energy to mechanically puncture the stratum corneum to create a micropore having a first depth.

The creation of the micropore disrupts at least one cell of at least the stratum corneum of the tissue surrounding the micropore and stimulates the tissue to provoke an immune response. The creation of the micropore disrupts the cells of the stratum corneum (or viable epidermis) of the tissue surrounding the micropore by displacing proteins (e.g., extra-cellular shock proteins) found within the cell. In one envisioned embodiment, the micro-heater disrupts one or more cells of the stratum corneum (or the viable epidermis) of the tissue surrounding the micropore by imparting thermal energy into the micropore, the disruption forming an immune response.

The present disclosure also relates to a method for forming a micropore in a barrier, the method including the initial step of providing at least one micro-heater housing an ablation material disposed therein having a volume. The micro-heater is adapted to connect to a power supply component which is configured to supply energy to the micro-heater to activate the ablation material. The method also includes the steps of: activating the micro-heater to transfer thermal energy to the ablation material; heating the ablation material to a predetermined temperature sufficient to rapidly increase the volume of the ablation material such that the ablation material mechanically punctures a barrier to form a micropore; and activating at least one of the micro-heater and ablation material to implement a subsequent disruptive event, the subsequent disruptive event increasing the depth of the micropore through the barrier.

The method may also include the additional or alternate step of activating at least one of the micro-heater and ablation material to implement a subsequent disruptive event, the subsequent disruptive event stimulating an immune response in the tissue surrounding the micropore.

The invention is defined in claim 1. Any embodiment which is in contradiction to claim 1 is not part ot the invention.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is a schematic illustration of a microporation device;
FIG. 2 is another schematic illustration of the microporation device having a number of hollow posts with an ablation material in an interior space of the posts;
FIG. 3 is another schematic illustration of the microporation device having a number of metallic hollow posts with the ablation material in an interior space of the posts and an inductive coil forming a number of micropores;
FIG. 4 is a side view of the first stage microporation to a depth d;
FIG. 5 is a side view of the second stage microporation to another depth greater than depth d to the viable epidermis;
FIG. 6 is a schematic illustration of the microporation device at the pre-activation stage;
FIG. 7 is a schematic illustration of the microporation device of Fig. 6 at the initial microporation stage;
FIG. 8 is a schematic illustration of the microporation device of Fig. 7 at the secondary microporation stage;
FIG. 9 is a schematic illustration of another embodiment of microporation device at the secondary microporation stage with a chemical agent;
FIG. 10 is a schematic illustration of another embodiment of microporation device at the secondary microporation stage with thermal energy;
FIG. 11 is a schematic illustration of another embodiment of microporation device at the initial microporation stage with plural ablation materials in each post;
FIG. 11A is a close up view of a post of FIG. 11 with plural ablation materials;
FIG. 11B is a close up view of a post of FIG. 11A with a first ablation material forming the initial micropore;
FIG. 11C is a close up view of a post of FIG. 11B with a second ablation material forming the enlarged micropore through the viable epidermis; and
Fig. 12 is a schematic flow diagram show one method according to the present disclosure.

### DETAILED DESCRIPTION

Embodiments of the presently disclosed temperature system will be described herein below with reference to the accompanying drawing figures wherein like reference numerals identify similar or identical elements. In the following description, well-known functions or constructions are not described in detail to avoid obscuring the disclosure in unnecessary detail.

The present disclosure relates to a microporation device 10. The microporation device 10 relates to a method disclosed in International PCT Patent Application No. PCT/US05/19035 to Prausnitz, et al., with an international filing date of May 31, 2005.

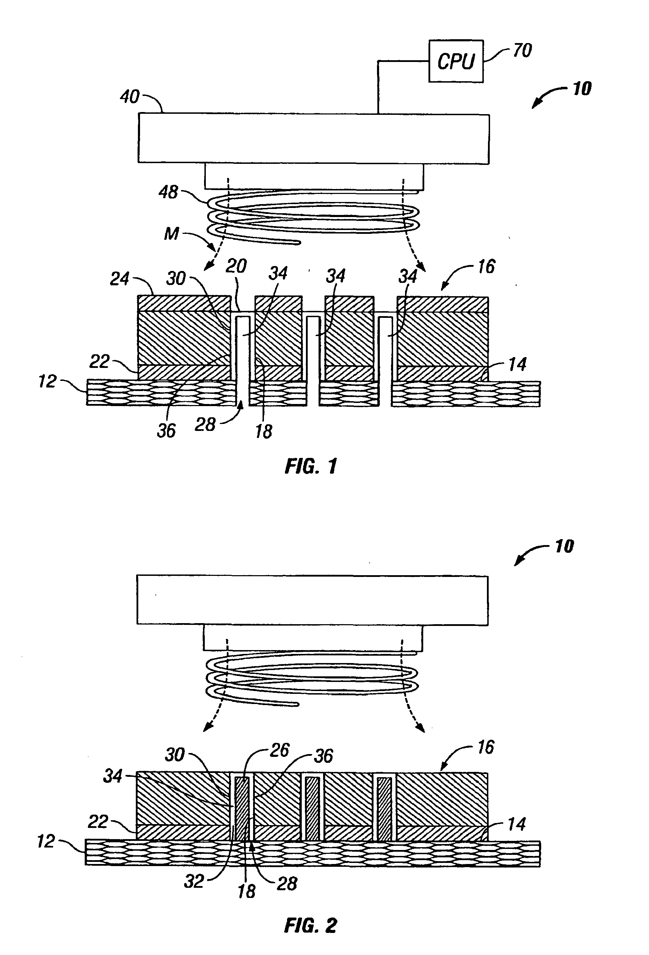

Referring now to Figs. 1 and 2, the microporation device 10 is configured to mechanically advance an active agent into a barrier 12 such as mammalian skin through the stratum corneum, viable epidermis and/or the dermis, or alternatively extract a material from the barrier 12. The viable or remaining epidermis of the barrier 12 lies below the stratum corneum. This viable or remaining epidermis layer of the barrier 12 is about ten times as thick as the stratum corneum; however, diffusion is much faster due to the greater degree of hydration in the living cells of the viable or remaining epidermis. The viable or remaining epidermis contains Langerhans' cells, which function as antigen-presenting cells to the immune system. The epidermis includes four histologically distinct layers which, from the inside to the outside, are the stratum germinativum, stratum spinosum, stratum granulosum, and the stratum corneum. The viable or remaining epidermis is meant to describe the underlying layers excluding the stratum corneum. The stratum corneum includes anucleated or dead cells and is the barrier to the delivery of an active agent.

The microporation device 10 is configured to operatively couple to one or more microprocessors 70 and includes one or more micro-heaters 16 each having a tip end 18 and a base 20. The tip end 18 is intended to contact the barrier 12 and be opposite the base 20. The microporation device 10 has the base 14 of the microporation device 10 being in contact with an insulation material 22 and a transdermal patch 24 having an active agent disposed therein. The microporation device 10 may be formed with the micro-heaters 16 made from one or more materials that are capable of being heated and then transferring heat to the surrounding environment. This material may be a single type of metal, layers of metal, conductive oxides, conductive polymers, or alloys, that include, but are not limited to, nickel, iron, ferromagnetic materials, copper, NiCu, PdCo, gadolinium-silicon-germanium alloy, aluminum, ceramic materials, electrodeposited materials, vapor deposited materials, gold, platinum, palladium outer layer coating of nickel, nickel iron, or a magnetic stainless steel alloy, indium tin oxide, lanthanum strontium cobalt oxide, and a aluminum doped zinc oxide.

The micro-heater 16 of the microporation device 10 is a hollow post 30 made from a thermal member with the tip end 18. The thermal member forming the post 30 has an interior space 34 and an outer surface 36. The interior space has an ablation material 26 that receives energy and undergoes a rapid phase change from one initial state to another second state.

For example, the phase change may be from a solid to a liquid. In another embodiment, the phase change may be from a liquid to a gas. In still another embodiment, the phase change of the ablation material 26 may be from a liquid having a first volume to a liquid having a second volume which is greater than the first volume. In still another embodiment, the ablation material 26 may undergo sublimation or a phase change from a solid to a gas. In one embodiment, the ablation material 26 is ethanol.

The ethanol is brought to a rapid temperature increase to increase a volume of the ethanol that will cause the ethanol to physically puncture the barrier 12, such as the stratum corneum forming a micropore 28 in the barrier 12 at a desired depth as shown in Fig. 1. Other and various micro heater 16 configurations are possible and within the scope of the present invention. In another embodiment, the ablation material 26 may be water, or water based solution. In still another embodiment, the ablation material 26 may be solid or a hydrated salt. Other examples include liquids, gels, solids, hydrophobic liquids, hydrophilic liquids, methanol, organic compounds, alcohols, ketones, aldehydes, amines, ethers, esters, oils, paraffins, fatty acids, salt hydrates, calcium hydrate, sodium sulphate, decahydrate, sodium phosphate, dodecahydrate, sodium phosphate dodecahydrate, calcium chloride hexahydrate, and sodium thiosulphate pentahydrate, mixtures or combinations.

The microporation device 10 further can be open facing the barrier 12 at the tip end 18 or have an insulating member 22 covering the hollow interior space 34 of the post 30. The insulating member 22 may be MYLAR™, KAPTON™ (polyimide), polyurethane, liquid crystal polymer, and epoxy or any material that provides thermal, chemical, or electromagnetic insulation, and is generally nonconductive or nonmagnetic. The microporation device 10 may have the thermal member of the post 30 in contact with the barrier 12 or not in physical contact with the barrier 12, and instead with the ablation material 26 in contact with the barrier 12. Alternatively, the insulating member 22 disposed on the tip end 18 of the thermal member (of the post 30) may be in contact with the barrier 12. Various configurations are possible and within the scope of the present disclosure.

The micro-heaters 16 having posts 30 may be any desired shape specific for the desired application. In one embodiment, it is envisioned that the micro-heaters 16 can be disk-shaped, cone-shaped, donut-shaped, loop-shaped or still other geometry such as orthogonal, and the size of the posts 30 can vary from one micron to hundreds of microns depending on the barrier 12 and the selected ablation material 26.

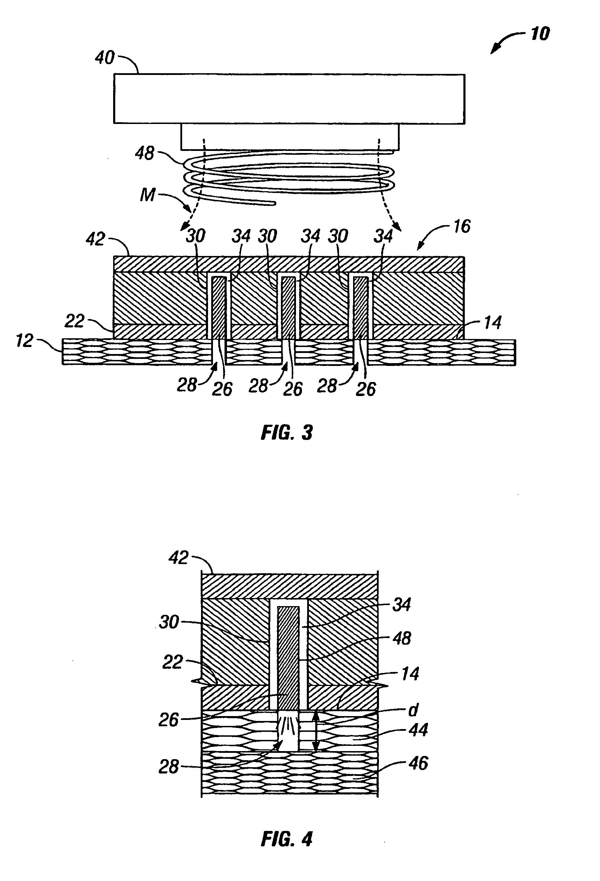

Referring now to Fig. 3 the microporation device 10 is typically connected to a power supply 40 and includes a substrate 42 and one or more micro-heaters 16 each having a thermal member in the form of a post 30 and a hollow interior 34 with an insulating material 22. An ablation material 26 such as an ethanol is disposed in the interior 34. The power supply 40 includes an alternating current energy supply component that is suitable to alter barrier permeability or to form micropores 28 in a barrier 12.

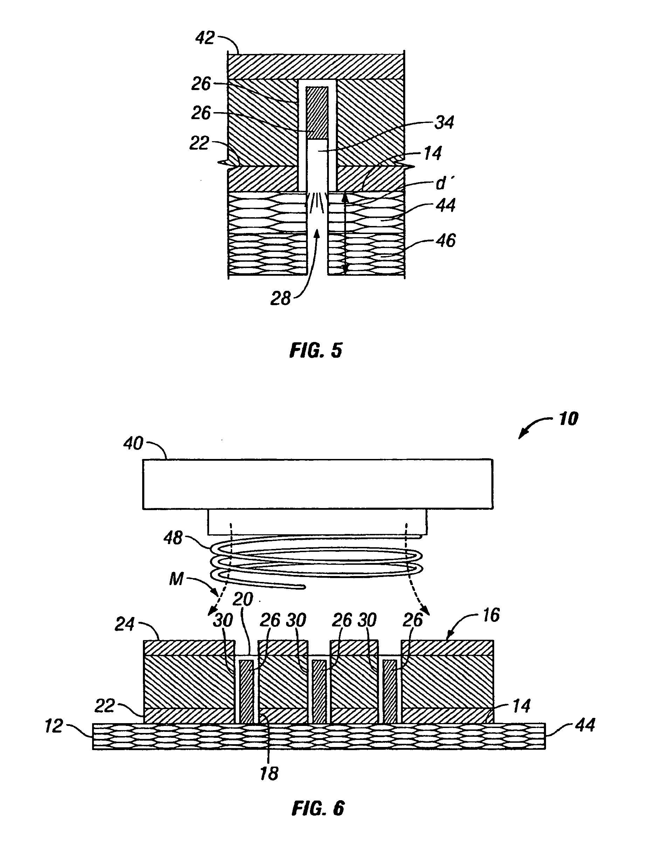

Referring now to Fig. 4, the microporation device 10 forms micropores 28 that are a sufficient depth through the stratum corneum 44 and through a portion of the epidermis 46 (such as in the case of the skin). The micropores 28 may have another depth for when the barrier 12 is a mucous membrane, or an inner covering such as a lining, a membrane or a surface of an organ or organ structure. The power supply 40 of Fig. 3 has sufficient power in order to increase barrier permeability by creating a first micropore 28 having a depth "d" sufficient to enter the stratum corneum 44, and then further increase barrier permeability by increasing the depth "d" of the first micropore 28 by a second disruptive event that increases the first depth "d' to a second depth "d' " as shown in Fig. 5. As used herein, the microporation device 10 may include any number of micro-heaters 16 in combination with the ablation material 26 that increases the permeability of the barrier 12, for example, by forming one or more micropores 28 in the barrier 12. An active agent from the device 10 is then introduced from the substrate 42 as shown in Figs. 3 and 5. It is envisioned that the substrate 42 has a compartment or a suitable absorbent patch and the active agent is pumped or diffused through the formed micropores 28 to the mammalian body. It should be appreciated that the second depth "d' " is not exactly twice the depth "d", and may be any depth known in the art that is simply greater than depth "d".

Referring again to Fig. 3, the power supply 40 of the microporation device 10 may include a wireless induction heating device for generating the micropores. This power supply component 40 is connected to an AC power source, and has an induction coil 48 which is electromechanically associated with one or more micro-heaters 16. The wireless energizing of the magnetic field M is indicated, and the base 14 of the device 10 is connected to the posts 30 that have the insulating material 22 connected thereto. The power supply component 40 comprises the coil 48, a radiofrequency generator, an amplifier and a controller (not shown) that are connected to a switch structure for controlling the coil 48. In this manner, AC current is run through the induction coil 48 to produce magnetic field M. These components are discussed in International PCT Patent Application No. PCT/US05/19035 to Prausnitz, et al., with an international filing date of May 31, 2005 which requires no further explanation.

Referring to Fig. 6, there is shown the pre-activation stage. The power supply components cause the energy to pass through the induction coil 48. The induction coil 48 emits the magnetic field "M" to heat the hollow posts 30 (or thermal member forming the posts 30). The posts 30 have the ablation material 26 contained therein. The micro-heaters 16 are heated to a desired temperature to create the micropores 28 in the barrier 12. These temperatures range from a cooled temperature, ambient temperature or room temperature to about 90 degrees Centigrade. In another embodiment, the temperature may be over about 400 degrees Centigrade while the barrier 12 is sufficiently not in contact with the micro-heaters 16 to not be heated above about 100 degrees Centigrade. In still another embodiment, the temperature of the posts 30 may be over about 400 degrees Centigrade while the barrier 12 is sufficiently insulated with the insulating material 22 so the micro-heaters 16 cannot heat the barrier 12 above about 100 degrees Centigrade. It should be appreciated that the thermal members of posts 30 are heated by inductive heating. In one embodiment, the thermal members at the tip end 18 of the posts 30 have a diameter of 400 microns and the base diameter 20 of 80 microns with a metal thickness of 50 microns and a total height of 2 mm. The tip diameter has a flat end for receipt of the insulating material 22 and does not have any sharp tip that is sufficient to puncture the barrier 12. It is envisioned that the posts 30 may be heated by other or alternative heating elements or systems including, ohmic or resistive heating mechanisms disposed proximate or adjacent the micro-heaters 16, microwave energy systems, by passing electrical current through the micro-heater 16, by passing electrical current across the interior space 34 of the micro-heater 16. For the explanational purposes of this disclosure, the posts 30 are described as being heated by inductive heating coils.

In one embodiment, the power supply 40 has an input current to the coil of about 1 amp, and the resulting heating power is about 1 Watt, and 8 Watts at 200 kHz, and 2 MHz, respectively. The microporation device 10 of the present disclosure occurs advantageously in two or more discrete stages. The device 10 uses the multiple stages in order to create relatively deep and narrow entry into the barrier 12 and internal disruption of the barrier 12 such as the stratum corneum.

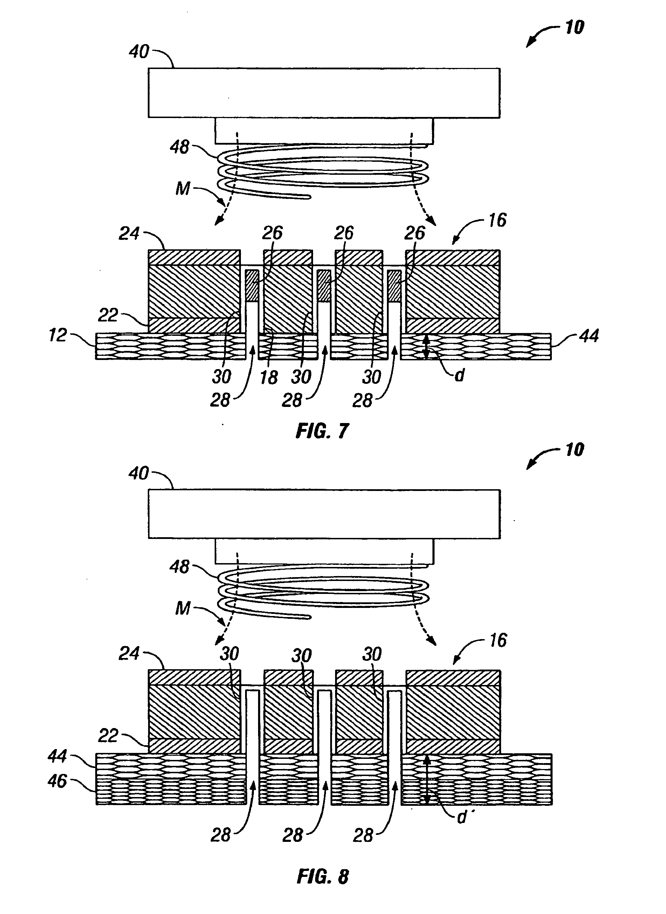

The microporation device 10 commences operation with a first disruptive event. The first disruptive event may be from one or more different microporation events that relate to puncturing the stratum corneum. The activation of the first disruptive event as discussed above is shown in Fig. 6. Fig. 7 shows the microporation device 10 in the initial first disruptive event. In this embodiment, the microporation device 10 includes power supply 40 with inductive coil 48 operatively connected to the radiofrequency generator. The microporation device 10 includes a number of hollow posts 30 formed in an array along the barrier 12 (e.g., 20 x 20 arranged in a generally orthogonal manner) with each of the posts 30 having the ablation material 26 disposed in the interior 34 of the post 30. In one embodiment, the hollow posts 30 have the liquid ethanol being disposed therein. The hollow posts 30 also have the insulating material 22 with a thickness being disposed at or adjacent to the tip end 18 of the hollow posts 30. The inductive coil 48, upon being energized with current, heats the hollow posts 30 in a rapid manner. In one envisioned embodiment, the hollow posts 30 reach a temperature of about 400 degrees Centigrade which causes the ethanol to rapidly increase in volume and move into and puncture the barrier 12 as shown.

Fig. 7 shows the creation of the initial micropore 28. A rapid increase of the volume of the ethanol or other ablation material 26 causes the ethanol to increase in volume and expand outward from the hollow post 30 in a direction toward the barrier 12. The ablation material 26 will contact the barrier 12 forming the micropore while the microporation device 10 has sufficient insulation 22 in order to keep the temperature of the barrier 12 under about 100 degrees Centigrade at all times. It should be appreciated that the thermal members at the tip end 18 of the posts 30 do not thermally induce the ablation of the barrier 12. It should be appreciated that the ablation of the barrier 12 is not caused by any thermal conduction between the posts 30 and the barrier 12, and instead the micropore is formed by the ablation material 26 mechanically puncturing the barrier 12.

It should further be appreciated that depth of the micropore in the barrier has a first depth "d" between about 10 to about 30 micrometers which, preferably, extends through the stratum corneum 44 as shown in Fig. 4 with a diameter of the micropore or region of increased permeability being complementary to that of the hollow post 30 and the thermal member. It is envisioned that for some embodiments and for the application of some therapeutic agents this depth "d" is sufficient such that the transdermal patch 24 may contain a therapeutic agent which can be introduced through the micropore 28 having the depth "d". However, for other embodiments introducing other therapeutic agents, a depth greater than about 10 to about 30 micrometers is desired. It is also envisioned that the references to "diameter" for the micropore 28 is not limiting and that other non-circular, non-cylindrical or non-tubular micropores 28 may be formed and are within the scope of the present disclosure. Also, the initial micropore formed may include more than one pathway through the stratum corneum 44 and collectively these pathways are considered a micropore 28 embodied in an indistinct shape. It should be appreciated that the disruptive event or mechanical puncturing may include any event that provides for a lipid bilayer disruption or a disorganization of the lipid bi-structure of a layer of the viable epidermis. The so-called disruptive event may also include any indiscriminate separation of the region of the viable epidermis to create a pathway to introduce an agent or for fluid extraction. Various micropore 28 configurations are possible.

Fig. 8 shows the microporation device 10 in the second stage. In the second stage, the microporation device 10 enlarges the depth "d" of the initial depth to a second depth "d' " greater than the first depth "d" in order to provide for a narrower deeper micropore 28 relative to the prior art. As can be appreciated, the mechanism for enlarging the depth "d" of the micropore 28 may include a thermally activated device, a mechanically-activated device or a chemically-activated device. Preferably, the diameter of the micropore remains substantially the same.

The second event expands the depth of penetration of the micropore 28 beyond the stratum corneum 44 to an enhanced depth "d' ", e.g., the second event allows fluid communication into viable epidermis layer 46 (or a layer beyond the stratum corneum 44 as shown in Fig. 5). In one embodiment, the second depth "d'" of the micropore 28 after the second stage is greater than about 30 micrometers. In another embodiment, the second depth "d' " of the micropore 28 after the second stage is greater than about 40 micrometers. In still another embodiment, the second depth "d' " after the second stage is in a range that includes about 40 micrometers to about 60 micrometers. In yet a further embodiment, the second depth "d' " is greater than about 60 micrometers. In yet a further embodiment, the second depth "d' " after the second stage is substantially the same as the initial depth "d" and is greater than about 30 micrometers with the second event producing an improvement in at least one relevant physiological property of the micropore. Various configurations are possible and it is envisioned that the second depth "d' " can have any depth that is dependent upon a number of factors including the specific active agent that is desired to be introduced into the body, the ablation material 26, the configuration of the post 30, and the energy supplied from the power supply 40.

Fig. 5 shows the second stage of the microporation device with the second depth "d' " of the micropore 28 being in the viable epidermis 46 beyond the stratum corneum layer 44. The second microporation event is an event that provides energy in order to disrupt the viable epidermis 46 beyond the stratum corneum layer 44 without excessively damaging or excessively enlarging the diameter of the initial micropore 28 of Fig. 7 beyond a predetermined limit.

In a first embodiment as shown in Fig. 8, the inductive heating coil 48 may further heat the ablation material 26 in order to further expand the ablation material 26 and further mechanically advance the ablation material 26 into the micropore 28 thereby increasing the depth "d' " of the micropore beyond the stratum corneum 44 and/or for producing an improvement in at least one relevant physiological property of the micropore 28. In another embodiment, the post 30 may include a first ablation material suitable 26 for forming the micropore 28 with the depth "d" and then a second ablation material 26' suitable for forming the enhanced or second depth "d' " and/or for producing an improvement in at least one relevant physiological property of the micropore 28. The first and the second ablation materials 26, 26' may both be disposed in the hollow post 30 of the microporation device 10, e.g., in a layered fashion

The microporation device 10 also may further heat the ablation material 26 to provoke an immune system response from the cells surrounding the micropore 28. Alternatively, the ablation material 26, itself, once released into the micropore 28 may provoke the immune system response. The thermal energy E or the ablation material 26 may rupture cells and spill out the contents of the cells into the surrounding areas to provoke an immune response. It has been observed that thermal energy E or the ablation material 26, once imparted to the cells of the viable epidermis 46 and/or the stratum corneum 44 after the formation of the micropore 28, are sufficient to displace proteins normally found inside cells creating extra cellular proteins. Once the extra cellular proteins are displaced in the surrounding areas, the extra cellular proteins will activate the immune system. The specific proteins that activate an immune system response are known to those skilled in the art as so-called "heat shock proteins". These heat shock proteins, which are bound to various peptides, perform functions in various intra-cellular processes during non-stressful conditions. Typically, the production of high levels of heat shock proteins occurs when cells are exposed to environmental stress conditions which may include infection, inflammation, toxins, adverse heat or cold, hypoxia or any condition that may contribute to or exacerbate cell death. In one envisioned embodiment of the present disclosure, the release of heat shock proteins concomitant with pathogens or infectious agents can promote the activation of innate and adaptive immunity.

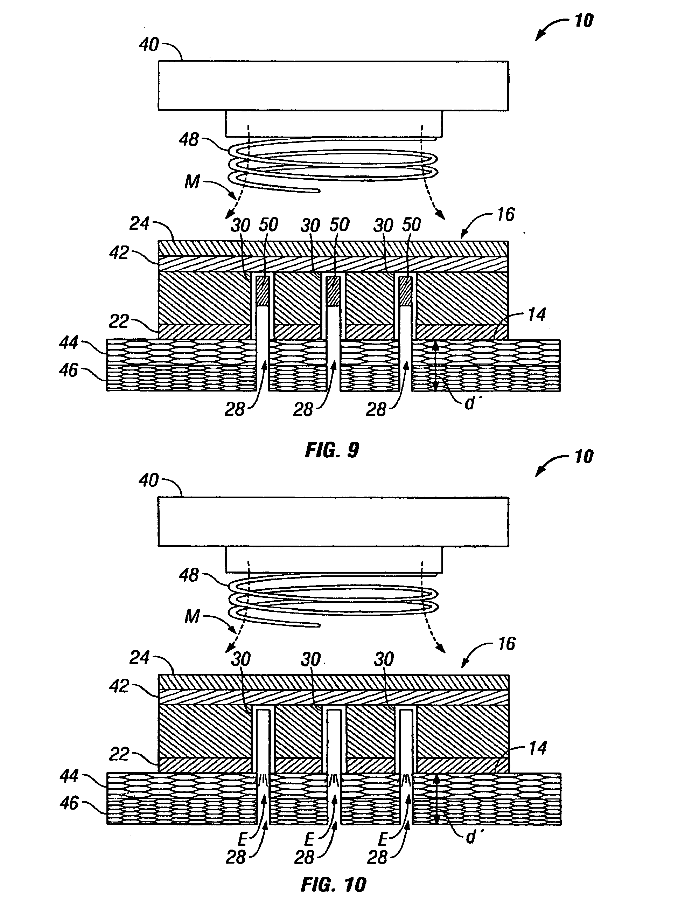

In another exemplary embodiment shown in Fig. 9, the secondary disruptive event may be attributed to an enlargement of the micropore 28 and/or produce an improvement in at least one relevant physiological property of the micropore 28 from a chemical agent 50 or substance that is different from, or the same as, the ablation material 26. The chemical agent 50 can be introduced after the formation of the micropore 28 from the hollow post 30, or subsequently after the formation of the micropore 28. As mentioned above, the chemical agent 50 may be introduced from the substrate 42, the hollow post 30, or an absorbent layer being disposed closely adjacent to the therapeutic agent, on the patch 24 or from another separate location relative to the microporation device 10. The chemical agent 50 may be introduced or moved from a first initial location and through the micropore 28.

The chemical agent 50 can be an organic compound or an inorganic compound that is the same as or different from the ablation material 26. The chemical agent 50 may be an organic acid or non-toxic corrosive substance, among many other substances, which undergoes a chemical and/or physiological reaction with the stratum corneum 44 and/or the viable epidermis 46 in order to porate the stratum corneum 44 and viable epidermis 46 to the second depth "d' " and/or produce an improvement in at least one relevant physiological property of the micropore in the second stage. In another embodiment, the chemical agent 50 may simply disrupt the stratum corneum 44 or the viable epidermis 46 to allow the active agent to enter the viable epidermis 46. Some other examples of the chemical agent 50 may include any organic or inorganic compound that can react with, provide a catalytic reaction with, or promote diffusion of the active agent into the micropore 28 and into the viable epidermis 46.

In one embodiment, the chemical agent 50 can be ethanol, or another cell toxin, such as, trace metals, or arsenic. In another embodiment, the chemical agent 50 can be any substance that places stress on the cells and regulates when the cells will rupture or spill out contents of the cell to provoke an immune response. The chemical agent 50 can be any substance that deprives the cells from oxygen to provoke an immune response, or any material that can promote high levels of heat shock proteins to be released from the cells to provoke the immune response. In one embodiment, the chemical agent 50 can be released at a depth where Langerhans cells are present in the body.

In another exemplary embodiment as shown in Fig. 10, the secondary disruptive event may be thermal energy E from the inductive coil 48 and or the hollow post 30 after the expansion of the ablation material 28. The thermal energy E can be introduced from the hollow post 30 after the formation of the micropore 28 where the hollow post 30 would impart a quantity of thermal energy E from the hollow post 30 and into the micropore 28 for increasing the depth "d' " of the micropore 28 and/or for producing an improvement in at least one relevant physiological property of the micropore. As mentioned, the thermal energy E may be introduced from another location exclusive from the hollow post 30 such as from another coil or another discrete heating device. The thermal energy E may be introduced from the separate heating device and transferred from conduction or convection (from contact with the source) from a first initial location and through the micropore 28.

The thermal energy E may be directly applied to the micropore 28 in order to increase a depth "d" of the micropore or the thermal energy E can be transferred to the ablation material 28 (in the second stage), or another organic compound or inorganic compound that is the same as or different from the ablation material 28 and that is non-toxic. The second heated material may be sequentially introduced into the micropore 28. The thermal energy E that is directly or indirectly applied to the micropore 28 contacts the surrounding lateral surfaces of the stratum corneum 44 and/or the viable epidermis 46 to enlarge the depth "d" on in order to porate to the second depth "d' " in the second stage. In another embodiment, the thermal energy E can simply disrupt the stratum corneum 44, the viable epidermis 46, or other cells to assist the active agent entering the viable epidermis 46 at an increased rate of diffusion. Some examples may include heating the surrounding lateral surface to temperatures in excess of about 100 degrees Celsius in order to ablate and increase the depth of the micropore 28 to the second depth "d' ". Various other temperature gradients are possible and within the scope of the present disclosure.

In another exemplary embodiment, the secondary disruptive event may be thermal energy E from the inductive coil 48 and or the hollow post 30 after the rapid expansion of the ablation material 26 whereas the temperature increase is significantly less than about 100 degrees Celsius. In this embodiment, the temperature is not high enough to ablate the tissue, but will promote an increase immune response. The thermal energy E can be introduced from the hollow post 30 after the formation of the micropore 28 where the hollow post 30 would impart a quantity of thermal energy from the hollow post 30 into the micropore 28, but not for increasing the depth of the micropore 28, and only for releasing proteins from the surrounding cells of the surrounding tissue of the micropore 28.

It has been observed that thermal energy E, as well as other environmental stress conditions, once imparted to the cells of the viable epidermis 46 and/or the stratum corneum 44 after the formation of the micropore 28, is sufficient to displace proteins normally found inside cells creating extra cellular proteins. The proteins once displaced will activate the immune system. The proteins thus provoke an immune response in that these proteins with their peptide complexes are detected by circulating immune system cells called antigen presenting cells. These proteins in the relevant cells of the viable epidermis and the stratum corneum that are displaced are called "heat shock" proteins or stress proteins. Once the antigen presenting cells of the lymphatic system haven taken in the heat shock proteins complexes, they travel to the lymph nodes and an immune response is stimulated that can facilitate introducing the active agent from the patch 24 or compartment. Such stimulation of the immune system may include stimulation of white blood cells that can include lymphocytes, monocytes, neutrophils, eosinophils, basophils, or any combination thereof. In one alternative embodiment, toxins or ultraviolet light may be released or emitted into the micropore 28 to kill or damage a predetermined number of cells to provoke the immune response.

As mentioned, the thermal energy E may be introduced from the hollow posts 30 and subsequent to the formation of the micropore 28. The thermal energy E may be introduced for a predetermined period of time or in a cycling arrangement that has a suitable maximum temperature to displace the proteins. In another embodiment, the microporation device 10 may further comprise an additional heating device such as a heating coil that is located at another location exclusive from the hollow posts such as closely adjacent the induction coil 48, the substrate 42 or near the active agent in the patch 24. The thermal energy E to displace the proteins may be introduced from the separate heating device and transferred from conduction with direct contact on the micropore 28 or convection from a first initial location and through the air, liquid or gas that surrounds micropore 28. The thermal energy E has a suitable maximum temperature to displace the proteins. In another embodiment, the thermal energy E can be transferred to the ablation material 26, or another non-toxic organic compound or inorganic compound that is the same as or different from the ablation material 26 that is introduced into the micropore 28.

The thermal energy E to displace the cellular proteins (that is directly or indirectly applied to the micropore 28) contacts the surrounding lateral tissue surfaces of the stratum corneum 44 and/or the viable epidermis 46. The energy E displaces at least some proteins. The displacement is of a magnitude in order to stimulate the immune system response in the second stage.

In another embodiment of the present disclosure, the microporation device 10 may include a second stage that includes a combination of disruptive events to increase the depth "d" of the micropore to the second depth "d' " and/or produce an improvement in at least one relevant physiological property of the micropore 28. In one embodiment, the micropore 28 may be formed at the first stage. At the second stage, a combination of chemical, thermal and/or mechanical disruptive events can increase the depth of the micropore from the first depth 'd" to the second depth "d' " and/or produce an improvement in at least one relevant physiological property of the micropore 28. In one embodiment, the second stage may include imparting thermal energy E to the ablation material 26 to further mechanically increase the depth of the micropore from the first depth "d" to the second depth "d' ". Thereafter, the microporation device 10 may then heat (e.g., by an inductive heating system, microwave heating system, ohmic or resistive heating systems or electrical system) the hollow posts 30 as shown in Fig. 10 so the hollow posts 30 impart thermal energy E to the micropore 28 assist the mechanical disruptive event from the first depth "d" to the second depth "d' "and/or produce an improvement in at least one relevant physiological property of the micropore 28.

In another embodiment, the micropore 28 may be formed at the first stage. The second stage may include imparting thermal energy E to the ablation material 26 to further ablate the tissue of the stratum corneum 44 and to increase the depth of the micropore 28 from the first depth "d" to the second depth "d' "and thereafter, the microporation device 10 may then introduce a chemical agent 50 as shown in Fig. 9 to react with tissue 44, 46, and increase the depth of the micropore from the first depth "d" to the second depth "d' "and/or produce an improvement in at least one relevant physiological property of the micropore 28.

In another embodiment, the second stage may include imparting thermal energy E to the ablation material 26 to further ablate the tissue of the stratum corneum 44 to increase the depth of the micropore 28 from the first depth "d" to the second depth "d' ". Thereafter, the microporation device 10 may then transfer thermal energy E at a temperature that is insufficient to ablate the tissue, but sufficient to displace the cellular proteins to initiate an immune response and to assist with introducing the active agent from the device 10 to the body.

In still another further embodiment, the second stage may include plural disruptive events. In this embodiment, the microporation device 10 may impart thermal energy E (as shown in Fig. 10) to the ablation material 26 to further ablate the tissue of the stratum corneum 44 to increase the depth of the micropore from the first depth "d" to the second depth "d' ", then introduce a chemical agent 50 (as shown in Fig. 9) to assist with increasing the depth of the micropore 28 from the depth "d" to the second depth "d' " (not shown) and further the microporation device 10 may then transfer thermal energy E at a temperature that is sufficient to ablate the tissue 44, 46 and/or produce an improvement in at least one relevant physiological property of the micropore 28. It should be appreciated that depth d is less than the second depth d' and the second depth d' is also less than the third depth d". Various configurations are possible and it is envisioned that any combination of plural disruptive events may be used with the microporation device 10 of the present disclosure that are sufficient to ablate, enlarge the micropore 28 or displace the cellular proteins to initiate an immune response and/or assist with introducing the active agent from the device 10 to the body at a greater flow rate. The micropore 28 may be formed from a number of events to achieve a predetermined depth.

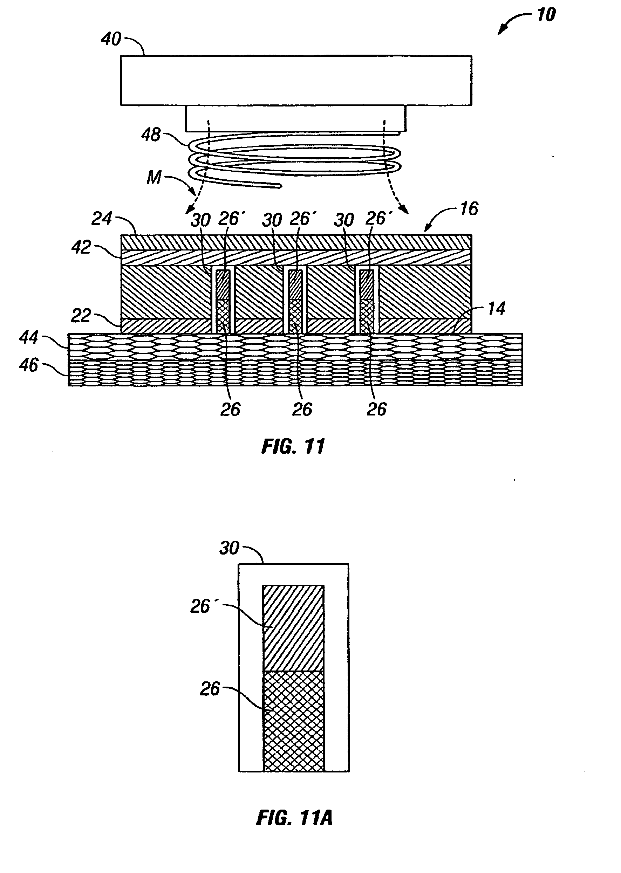

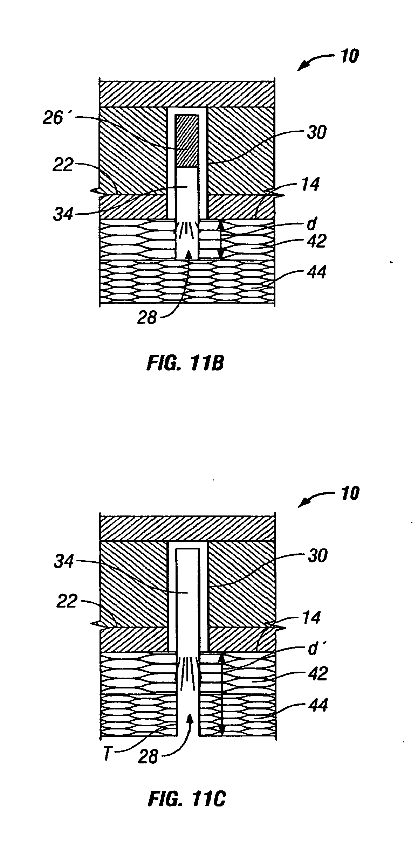

Referring now to Fig. 11, there is shown another embodiment of the microporation device 10. In this embodiment, the microporation device 10 has a substrate 42, and a layer 24 for containing the active agent, and an inductive coil 48 connected to the generator (not shown). The microporation device 10 also has a number of hollow posts 30. Referring to an enlarged view shown as Fig. 11A, each of the hollow posts 30 has a plurality of ablation materials 26, 26' being disposed therein. Each of the ablation materials 26, 26' has a predetermined rate of volume expansion relative to the desired microporation stage. In one embodiment, the hollow post 30 has a first ablation material 26 that will rapidly expand at a first temperature T. The hollow post 30 also has a second ablation material 26' that will rapidly expand at a second temperature T' that is greater than the first temperature T.

In this manner, as the inductive coil 48 heats the hollow post 30, and the first ablation material 26 reaches the first temperature T, the first ablation material 26 will rapidly expand and form the micropore having the first depth "d". At this initial stage, the second ablation material 26' being heated to a first temperature T will remain dormant and in an initial phase. The second ablation material 26' may remain outside the micropore 28 for a period of time, or alternatively may enter the micropore 28. However, as the temperature of the hollow posts 30 increases from being heated by the inductive heating, the temperature of the second ablation material will rise from the first temperature T to the second temperature T'.

The first ablation material 26 and the second ablation material 26' may be two completely different inorganic or organic materials that are non-toxic or the second ablation material 26' may be a derivation of the ablation material 26. For example, the first ablation material 26 may be an ethanol and the second ablation material may be an ethanol with a number of molecules or a compound that contributes with the later disruption. Various configurations are possible and within the present disclosure.

Referring to Fig. 11C, upon reaching, the second temperature T', the second ablation material 26' will undergo a volumetric increase or phase change from the initial phase to the second phase. This will cause the second ablation material 26' to mechanically increase the depth of the micropore from the initial depth "d° to a second depth "d' " with the second depth "d' " being greater than the depth "d" for a narrower deeper micropore 28.

In another embodiment, the second ablation material 26' may not rapidly change in volume. The second ablation material 26' may instead chemically react with the surrounding lateral tissue of the micropore T (shown In Fig. 11 C) to assist with the material 26' chemically reacting with the stratum corneum 42 and/or the viable epidermis 44 in order to increase the micropore 28 from the initial depth "d" to a second depth "d' " and/or produce an improvement in at least one relevant physiological property of the micropore 28. As understood, the second ablation material 26' may be any material for enlarging to the second depth "d' " with the second depth "d' " being greater than the depth "d" for a narrower deeper micropore 28.

According to another embodiment of the present disclosure, the microporation device 10 may further create an initial micropore 28 for fluid extraction rather than introducing agents into the micropore 28. In this manner, it is contemplated that the microporation device 10 may cause plural disruptive events through the stratum corneum 44 and remaining epidermis 46 as discussed above. In this manner, the microporation device 10 increases a depth of the micropore 28 for increased fluid extraction from the remaining viable epidermis 46 to readily extract an analyte for testing purposes. It is envisioned that the microporation device 10 can harvest fluid from the tissue for analyte measurement, ph measurement, glucose measurement, or any other analyte in the mammalian body. The microporation device 10 may use a vacuum or other device to create a gradient to extract fluid from the micropore 28. In one embodiment, the gradient may be a pressure gradient.

In still yet another embodiment, the present disclosure also relates to a method for forming a micropore in a barrier. The method includes the initial step of providing at least one micro-heater housing an ablation material disposed therein having a volume. The micro-heater is adapted to connect to a power supply component which is configured to supply energy to the micro-heater to activate the ablation material. The method also includes the steps of: activating the micro-heater to transfer energy to the ablation material; heating the ablation material to a predetermined temperature sufficient to rapidly increase the volume of the ablation material to form a micropore in tissue; introducing at least one anti-healing agent into the micropore to maintain the diffusivity of the tissue; and activating at least one of the micro-heater and ablation material to implement a subsequent disruptive event.

As can be appreciated, it is envisioned that the step of introducing at least one anti-healing agent into the micropore to maintain the diffusivity of the tissue may be included before or after the first or activating events to maintain the diffusivity of the tissue. Anti-healing agent may include any one or combination of the following substances: sodium chloride, calcium-based salts, anti-coagulating agents such as heparin, ethylenediaminetetraacetic acid (EDTA), citric acid, citrate salts, antiinflammatory substances such as hydrocortisone and combination thereof.

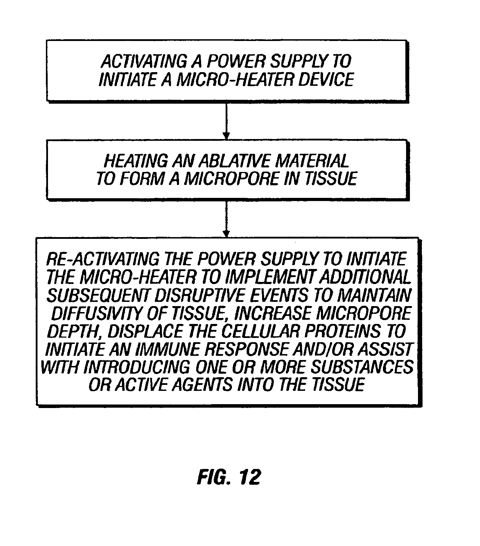

Fig. 12 shows another envisioned method according to the present disclosure which includes the steps of: activating a power supply to initiate a micro-heater device; heating an ablative material to form a micropore in tissue (e.g., by heating the ablation material to a predetermined temperature sufficient to rapidly increase the volume of the ablation material to form a micropore in tissue); re-activating the power supply to initiate the micro-heater to implement additional subsequent disruptive events to accomplish a particular purpose or to achieve a particular result, maintain diffusivity of tissue, increase micropore depth, displace the cellular proteins to initiate an immune response and/or assist with introducing one or more substances or active agents as described above.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

## Claims

1. A thermal treatment device (10) for forming a micropore (28) in a barrier, comprising:
at least one micro-heater (16) having a base end (20) and at least one post (30) defined therein, the post (30) including an interior volume for housing an ablation material (26) therein;
a power supply component (40) operatively associated with the at least one micro-heater (16), the power supply component (40) being configured to supply energy to the micro-heater (16) to activate the ablation material (26) such that the ablation material (26)expands in response to the energy to mechanically puncture the barrier (12) to create a micropore (28) having a first depth (d); wherein the device is a multi-stage microporation device, **characterized in that** the interior volume of the post (30) includes a chemical agent (50) different from the ablation material (26)
wherein the chemical agent (5) is provided for chemically reacting with the barrier (12) to enlarge the micropore (28) to a second depth (d').

2. The thermal treatment device (10) according to claim 1, wherein the chemical agent (50) is adapted to stimulate a tissue response by the enlargement of the depth of the micropore (28) to the second depth (d').

3. The thermal treatment device (10) according to any preceding claim, wherein the at least one post (30) includes a plurality of different materials each having a different thermal conductivity which activates the ablation material (26) at different stages to increase the micropore depth at correspondingly different stages.

4. The thermal treatment device (10) according to any preceding claim, wherein the ablation material (26) includes ethanol and wherein the micro-heater (16) includes an insulator material (22) which prevents excessive heating of the barrier (12) during activation of the ablation material (26).

5. The thermal treatment device (10) according to claim 4, wherein the insulator material (22) prevents the barrier (12) from reaching about one hundred degrees Centigrade.

6. The thermal treatment device (10) according to any preceding claim, wherein the first depth (d) is about ten microns to about thirty microns, and wherein the second depth (d') is greater than about thirty microns.

7. The thermal treatment device (10) according to any preceding claim, wherein the barrier is skin, and wherein the first depth (d) is in a range of about ten microns to about thirty microns, and wherein the second depth (d') is sufficient to extend beyond thirty microns into the viable epidermis.

8. The thermal treatment device (10) according to any preceding claim wherein the chemical agent (50) disrupts at least one cell of the epidermis of the tissue surrounding the micropore (28), the disruption stimulating an immune response from the tissue.

9. The thermal treatment device (10) according to any preceding claim, wherein the chemical agent (50) disrupts at least one cell of the epidermis of the tissue surrounding the micropore (28) by displacing proteins found within the cell.

10. The thermal treatment device (10) according to claim 9, wherein the proteins are heat shock proteins.

11. The thermal treatment device (10) according to any preceding claim, wherein the ablation material (26) includes ethanol.

12. The thermal treatment device (10) according to any preceding claim, wherein the micro-heater (16) disrupts at least one cell of the barrier (12) surrounding the micropore (28) by imparting thermal energy into the micropore (28), the disruption forming an immune response.

## Patentansprüche

1. Wärmebehandlungseinheit (10) zum Bilden einer Mikropore (28) in einer Barriere, umfassend:
wenigstens einen Mikroheizer (16) mit einem Fußende (20) und wenigstens einer darin definierten Säule (30), wobei die Säule (30) ein inneres Volumen zum Aufnehmen eines Ablationsmaterials (26) darin aufweist;
eine Stromversorgungskomponente (40) in operativer Verbindung mit dem wenigstens einen Mikroheizer (16), wobei die Stromversorgungskomponente (40) dafür gestaltet ist, den Mikroheizer (16) mit Energie zu versorgen, um das Ablationsmaterial (26) zu aktivieren, so dass sich das Ablationsmaterial (26) in Antwort auf die Energie ausdehnt, um die Barriere (12) mechanisch zu durchstoßen, um eine Mikropore (28) mit einer ersten Tiefe (d) zu erzeugen; wobei die Einheit eine mehrstufige Mikroporationseinheit ist, **dadurch gekennzeichnet, dass** das innere Volumen der Säule (30) ein chemisches Mittel (50) enthält, das von dem Ablationsmaterial (26) verschieden ist;
wobei das chemische Mittel (5) zur chemischen Umsetzung mit der Barriere (12) bereitgestellt ist, um die Mikropore (28) auf eine zweite Tiefe (d') zu vergrößern.

2. Wärmebehandlungseinheit (10) gemäß Anspruch 1, wobei das chemische Mittel (50) dafür ausgelegt ist, durch die Vergrößerung der Tiefe der Mikropore (28) auf die zweite Tiefe (d') eine Gewebeantwort anzuregen.

3. Wärmebehandlungseinheit (10) gemäß einem der vorstehenden Ansprüche, wobei die wenigstens eine Säule (30) eine Vielzahl verschiedener Materialien enthält, von denen jedes eine andere Wärmeleitfähigkeit aufweist, die das Ablationsmaterial (26) bei verschiedenen Stadien aktivieren, um die Tiefe der Mikropore bei entsprechend verschiedenen Stadien zu erhöhen.

4. Wärmebehandlungseinheit (10) gemäß einem der vorstehenden Ansprüche, wobei das Ablationsmaterial (26) Ethanol enthält und wobei der Mikroheizer (16) ein Isolatormaterial (22) enthält, das übermäßiges Erhitzen der Barriere (12) während der Aktivierung des Ablationsmaterials (26) verhindert.

5. Wärmebehandlungseinheit (10) gemäß Anspruch 4, wobei das Isolatormaterial (22) verhindert, dass die Barriere (12) etwa einhundert Grad Celsius erreicht.

6. Wärmebehandlungseinheit (10) gemäß einem der vorstehenden Ansprüche, wobei die erste Tiefe (d) etwa zehn Mikrometer bis etwa dreißig Mikrometer beträgt und wobei die zweite Tiefe (d') mehr als etwa dreißig Mikrometer beträgt.

7. Wärmebehandlungseinheit (10) gemäß einem der vorstehenden Ansprüche, wobei die Barriere Haut ist und wobei die erste Tiefe (d) im Bereich von etwa zehn Mikrometer bis etwa dreißig Mikrometer liegt und wobei die zweite Tiefe (d') ausreicht, um sich mehr als dreißig Mikrometer in die lebende Epidermis zu erstrecken.

8. Wärmebehandlungseinheit (10) gemäß einem der vorstehenden Ansprüche, wobei das chemische Mittel (50) wenigstens eine Zelle der Epidermis des Gewebes, das die Mikropore (28) umgibt, aufbricht, wobei das Aufbrechen eine Immunantwort des Gewebes anregt.

9. Wärmebehandlungseinheit (10) gemäß einem der vorstehenden Ansprüche, wobei das chemische Mittel (50) wenigstens eine Zelle der Epidermis des Gewebes, das die Mikropore (28) umgibt, aufbricht, indem es in der Zelle vorhandene Proteine verdrängt.

10. Wärmebehandlungseinheit (10) gemäß Anspruch 9, wobei die Proteine Hitzeschockproteine sind.

11. Wärmebehandlungseinheit (10) gemäß einem der vorstehenden Ansprüche, wobei das Ablationsmaterial (26) Ethanol enthält.

12. Wärmebehandlungseinheit (10) gemäß einem der vorstehenden Ansprüche, wobei der Mikroheizer (16) wenigstens eine Zelle der Barriere (12), die die Mikropore (28) umgibt, aufbricht, indem sie Wärmeenergie in die Mikropore (28) überträgt, wobei das Aufbrechen eine Immunantwort bewirkt.

## Revendications

1. Dispositif de traitement thermique (10) permettant de former un micropore (28) dans une barrière, comprenant :
au moins un micro-élément chauffant (16) ayant une extrémité de base (20) et au moins un montant (30) défini dedans, le montant (30) comportant un volume interne destiné à recevoir un matériau d'ablation (26) dedans ;
un composant d'alimentation électrique (40) associé de manière opérationnelle à l'au moins un micro-élément chauffant (16), le composant d'alimentation électrique (40) étant configuré pour alimenter le micro-élément chauffant (16) en énergie afin d'activer le matériau d'ablation (26) de sorte que le matériau d'ablation (26) se dilate en réponse à l'énergie pour percer mécaniquement la barrière (12) de manière à créer un micropore (28) ayant une première profondeur (d) ; où le dispositif est un dispositif de formation de micropores en plusieurs étapes, **caractérisé en ce que** le volume interne du montant (30) comporte un agent chimique (50) différent du matériau d'ablation (26)
dans lequel l'agent chimique (50) est fourni pour réagir chimiquement avec la barrière (12) afin d'agrandir le micropore (28) jusqu'à une deuxième profondeur (d').

2. Dispositif de traitement thermique (10) selon la revendication 1, dans lequel l'agent chimique (50) est adapté pour stimuler une réponse tissulaire par l'agrandissement de la profondeur du micropore (28) jusqu'à la deuxième profondeur (d').

3. Dispositif de traitement thermique (10) selon l'une quelconque des revendications précédentes, dans lequel l'au moins un montant (30) comporte une pluralité de matériaux différents ayant chacun une conductivité thermique différente qui active le matériau d'ablation (26) à des étapes différentes pour augmenter la profondeur de micropore à des étapes différentes correspondantes.

4. Dispositif de traitement thermique (10) selon l'une quelconque des revendications précédentes, dans lequel le matériau d'ablation (26) comporte de l'éthanol et où le micro-élément chauffant (16) comporte un matériau isolant (22) qui empêche un chauffage excessif de la barrière (12) pendant l'activation du matériau d'ablation (26).

5. Dispositif de traitement thermique (10) selon la revendication 4, dans lequel le matériau isolant (22) empêche la barrière (12) d'atteindre environ cent degrés Celsius.

6. Dispositif de traitement thermique (10) selon l'une quelconque des revendications précédentes, dans lequel la première profondeur (d) est d'environ dix microns à environ trente microns, et où la deuxième profondeur (d') est supérieure à environ trente microns.

7. Dispositif de traitement thermique (10) selon l'une quelconque des revendications précédentes, dans lequel la barrière est la peau, et où la première profondeur (d) se trouve dans une plage allant d'environ dix microns à environ trente microns, et où la deuxième profondeur (d') est suffisante pour s'étendre au-delà de trente microns dans l'épiderme viable.

8. Dispositif de traitement thermique (10) selon l'une quelconque des revendications précédentes, dans lequel l'agent chimique (50) rompt au moins une cellule de l'épiderme du tissu entourant le micropore (28), la rupture stimulant une réponse immunitaire au niveau du tissu.

9. Dispositif de traitement thermique (10) selon l'une quelconque des revendications précédentes, dans lequel l'agent chimique (50) rompt au moins une cellule de l'épiderme du tissu entourant le micropore (28) en déplaçant des protéines trouvées à l'intérieur de la cellule.

10. Dispositif de traitement thermique (10) selon la revendication 9, dans lequel les protéines sont des protéines de choc thermique.

11. Dispositif de traitement thermique (10) selon l'une quelconque des revendications précédentes, dans lequel le matériau d'ablation (26) comporte de l'éthanol.

12. Dispositif de traitement thermique (10) selon l'une quelconque des revendications précédentes, dans lequel le micro- dispositif de chauffage (16) rompt au moins une cellule de la barrière (12) entourant le micropore (28) en transmettant de l'énergie thermique dans le micropore (28), la rupture provoquant une réponse immunitaire.
